# EUROPEAN PATENT SPECIFICATION

(11) **EP 1 784 231 B1**
(45) Date of publication and mention of the grant of the patent: **04.08.2010**
(21) Application number: 05763864.5
(22) Date of filing: 29.06.2005
(51) Int. Cl.: A61L 9/03, A61L 9/12

(54) **ATOMISER WITH WICK HAVING NON-CAPILLARY CORE**
ZERSTÄUBER MIT DOCHT MIT NICHTKAPILLAREM KERN
ATOMISEUR AVEC UN NOYEAU DE MECHE NON-CAPILLAIRE

(30) Priority: 30.06.2004 US 583787 P
(43) Date of publication of application: 16.05.2007
(60) Divisional application: 10159855.5
(73) Proprietor: S.C. JOHNSON & SON, INC., Racine, Wisconsin 53403 (US)
(72) Inventor: VARANASI, Padma Prabodh, Racine, Wisconsin 53402 (US); ADAIR, Joel E., Racine, Wisconsin 53402 (US)
(74) Representative: Ruschke, Hans Edvard
(86) International application number: PCT/US2005/023229
(87) International publication number: WO 2006/004902

(56) References cited:
- EP-A- 0 423 816
- EP-A- 0 669 137
- EP-A- 0 897 755
- WO-A-96/07484
- GB-A- 2 357 973
- US-A1- 2004 065 749
- US-B1- 6 361 752

## Description

### BACKGROUND OF THE INVENTION

### 1. Field of the Invention

This invention relates to atomizer devices and wicks used for transporting liquids. Specifically, this invention relates to a wick-based delivery system for transporting liquids, such as fragrances or insecticides, from a reservoir to the ambient environment. The liquids are transported to the surface of an orifice plate which atomizes the liquid and ejects the atomized droplets into the ambient air.

### 2. Description of the Related Art

Devices that dispense vapors into the ambient air are well-known in the art and useful in many applications. Generally, the purpose of these devices is to deodorize or disinfect the ambient air, or to distribute toxins into the air to kill or repel unwanted pests.

A common method of dispersing vapors into the air utilizes a wick to deliver a vaporizable liquid from a reservoir to an exposed surface. The wick transports the liquid from the reservoir to the surface of the wick using capillary action. When the liquid reaches the surface of the wick, after migrating through porous material of the wick, it vaporizes and disperses into the air.

More recently, liquid atomizing devices have been used to disperse fragrances, insecticides, or the like into the ambient air. An example of such an atomizing device is shown in U.S. Patent No. 6,450,419 and in document EP 0897755 on which the pre-characterizing part of claim 1 is based. Often in atomization devices, a wick is used to convey liquid from a reservoir to an orifice plate. Vibration of the orifice plate atomizes the liquid and ejects the minute droplets into the ambient air.

However, when an atomizer device using fluid delivered by a wick is not activated, the capillary action of the wick may still be active and lead to migration of the liquid to parts of the atomizer. In such cases, an excess supply of liquid can flood the orifice plate and degrade the efficacy of the atomizer, when operated. To reduce such flooding, it is preferable to reduce the rate of fluid transfer through the wick to the orifices plate. Conventional methods of reducing the liquid flow rate through the wick, such as decreasing the porosity or changing the size of the wick, have drawbacks due to the preferred size and compressibility of the wicks for proper operation of an atomizer device. Accordingly, there is a need for as wick that can limit and control the flow rate of liquid from a reservoir to an orifice plate in an atomizer device, while still supplying a sufficient amount of liquid to the orifice plate for preferred atomization and meeting the preferred size and compressibility of the wick.

Document WO 96/07484 discloses a wick consisting of a tube with a non-porous outer wall and a convoluted inner wall forming a porous channel in the core of the wick.

### SUMMARY OF THE INVENTION

Our invention, which is defined in claim 1 addresses the problems mentioned above by improving the delivery of liquid through a wick. More specifically, our invention reduces the flow rate through the wick to reduce flooding, while maintaining a flow rate suitable for atomization, as well as the preferred size and rigidity of the wick.

The wick comprises a porous portion having capillary passages for drawing the liquid from a lower end to an upper end and a substantially non-porous portion that draws little or no liquid. The combination of a porous portion with a substantially non-porous portion allows for preferred control of the redaction of the flow rate of the liquid through the wick.

Most preferably, the non-porous portion is completely non-porous; however, as a practical matter, a portion of wick with no porosity may be difficult to achieve, given manufacturing limitations. The term non-porous will refer to wicks having a porosity of less than about five percent by volume. A more preferred wick will have a "non-porous" portion having a porosity of less than about four percent by volume. Even more preferably, the wick will have a non-porous portion-having a porosity of less than about three percent by volume. Even more preferably, the wick will have a non-porous portion having a porosity of less than about two percent by volume. More preferably, the wick will have a non-porous portion having a porosity of less than about one percent by volume. Most preferably, the will wick will have a non-porous portion that has a porosity of substantially zero percent by volume.

### BRIEF DESCRIPTION OF THE DRAWINGS

Figure 1 shows an elevational cross-section of an atomizer device embodying our invention.

Figure 2 shows a top view of a wick according to the preferred embodiment.

Figure 3 shows a cross-sectional view of the wick from Figure 2.

Figure 4 shows a top view of a wick according to another possible embodiment of the present invention.

Figure 5 is deleted.

Figure 6 shows a top view of a wick according to another possible embodiment of the present invention.

Figure 7 shows a cross-sectional, side view of another possible embodiment, which is not according to the present invention.

### DETAILED DESCRIPTION OF THE PREFERRED EMBODIMENTS

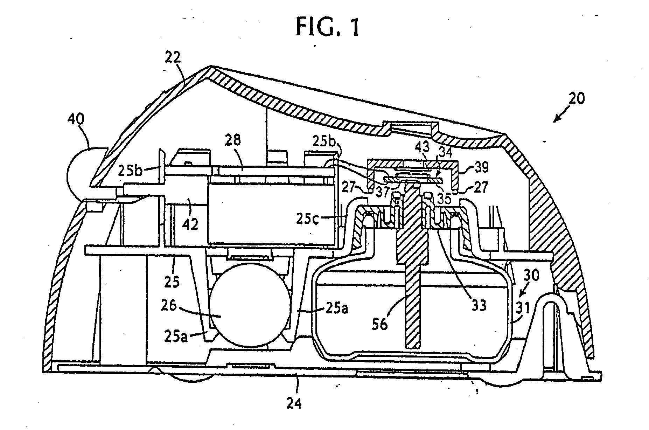

The preferred use of the wick described in this invention is for transporting liquid from a reservoir to an orifice plate in an atomization device. As shown in Figure 1, an atomization device 20 according to our invention typically comprises an atomizer assembly 34, which includes an orifice plate 37, and a replaceble reservoir assembly 30. The reservoir assembly 30 includes a reservoir 31 containing fluid and a wick 56.

The piezoelectrically actuated atomization device 20 according to a preferred embodiment of our invention comprises a housing 22 formed as a hollow plastic shell and closed by a flat bottom wall 24. A horizontal platform 25 extends across the interior of the housing 22. A battery 26 is supported by means of support prongs 25a which extend down from the underside of the platform 25 inside the housing 22. In addition, a printed circuit board 28 is supported on support elements 25b which extend upwardly from the platform 25. A liquid reservoir assembly 30 is replaceably mounted to the underside of a dome-like formation 25c on the platform 25.

The liquid reservoir assembly 30 comprises a liquid container 31 for holding a liquid to be atomized, a plug 33, which closes the top of the container, and the wick 56, which extends from within the liquid container 31 through the plug 33, to a location above the liquid container 31. The plug 33 is constructed to allow removal and replacement of the complete liquid reservoir assembly 30 from the underside of the dome-like formation 25c on the platform 25. Preferably, the plug 33 and the platform are formed with a bayonet attachment (not shown) for this purpose. When the replaceable liquid reservoir assembly 30 is mounted on the platform 25, the wick 56 extends up through a center opening in the dome-like formation 25c. The wick 56, which is described in greater detail hereinafter, operates by capillary action to deliver liquid from within the liquid container 31 to a location just above the dome-like formation 25c on the platform 25.

An atomizer assembly 34 is supported on the platform 25 in cantilever fashion by means of a resilient, elongated wire-like support 27. The wire-like support 27 is attached at the ends, which protrude upward from the platform 25. The wire-like support 27 is shaped such that it resiliently supports the lower surface of the orifice plate 37 and a spring housing 39, while a spring 43 resiliently presses on the upper surface of the orifice plate 37. (Rather than press on the orifice plate 37 itself, the spring 43 may alternatively or additionally press on a member; such as an actuator element 35, discussed below, which is connected to the orifice plate 37.) Together, the support 27 and the spring 43 hold the orifice plate 37 in place in a manner that allows the orifice plate 37 to move up and down against the resilient bias of the wire-like support 27.

The atomizer assembly comprises an annularly shaped piezoelectric actuator element 35 and the circular orifice plate 37, which extends across and is soldered or otherwise affixed to the actuator element 35. When alternating voltages are applied to the opposite upper and lower sides of the actuator element 35 these voltages produce electrical fields across the actuator element 35 and cause it to expand and contract in radial directions. This expansion and contraction is communicated to the orifice plate 37 causing it to flex so that a center region thereof vibrates up and down. The center region of the orifice plate 37 is domed slightly upward to provide stiffness and to enhance atomization. The center region is also formed with a plurality of minute orifices which extend through the orifice plate 37 from the lower or under surface of the orifice plate 37 to its upper surface. A flange is provided around the center region of the dome.

In operation, the battery 26 supplies electrical power to circuits on the printed circuit board 28 and these circuits convert this power to high frequency alternating voltages. A suitable circuit for producing these voltages is shown and described in U.S. Patent No. 6,296,196. As described in that patent, the device may be operated during successive on and off times. The relative durations of these on and off times can be adjusted by an external switch actuator 40 on the outside of the housing 22 and coupled to a switch element 42 on the printed circuit board 28.

When the atomizer assembly 34 is supported by the support member 27, the flange of the orifice plate 37 is positioned in contact with the upper end of the wick 56. The atomizer assembly 34 is thereby supported above the liquid reservoir assembly 30 such that the upper end of the wick 56 touches the underside of the orifice plate 37. Thus, the wick 56 delivers liquid from within the liquid reservoir 31 by capillary action to the underside of the orifice plate 37, which upon vibration, causes the liquid to pass through its orifices and be ejected from its opposite side (i.e., the upper surface) in the form of very small droplets.

It will be appreciated from the foregoing that the horizontal platform 25 serves as a common structural support for both the liquid reservoir assembly 30 and the atomizer assembly 34. Thus, the horizontal platform maintains the liquid reservoir assembly 30, and particularly, the upper end of the wick 56, in alignment with the orifice plate 37 of the atomizer assembly 34. Moreover, because the atomizer assembly 34 and the orifice plate 37 are resiliently mounted, the upper end of the wick 56 preferably presses against the under surface of the orifice plate 37 and/or the actuator element 35 irrespective of dimensional variations which may occur due to manufacturing tolerances when one liquid reservoir is replaced by another. This is because if the wick 56 of the replacement liquid reservoir assembly 30 is higher or lower than the wick 56 of the original liquid reservoir assembly 30, the action of the spring 43 will allow the orifice plate 37 to move up and down according to the location of the wick 56 in the replacement reservoir assembly 30, so that the wick 56 will properly press against the underside of the orifice plate 37 and/or the actuator element 35. It will be appreciated that the wick 56 preferably is formed of a solid, dimensionally stable material so that it will not become deformed when pressed against the underside of the resiliently supported orifice plate 37.

The wick in the present invention consists of a porous section and a substantially non-porous section. The porous section transports the liquid from the reservoir to the orifice plate through capillary action and the substantially non-porous section impedes the flow of liquid through a portion of the wick. The size of the substantially non-porous section of the wick may be varied as necessary to reduce the flow rate of liquid enough to reduce flooding, but preferably not so much that the desired amount of liquid does not reach the orifice plate. The addition of the substantially non-porous section of the wick facilitates control of the flow rate through the wick while still maintaining a wick of the necessary size for operation of the atomizer device. Of course, a wick according to our invention may be used in other devices, other than atomization devices, to achieve preferred flow rates.

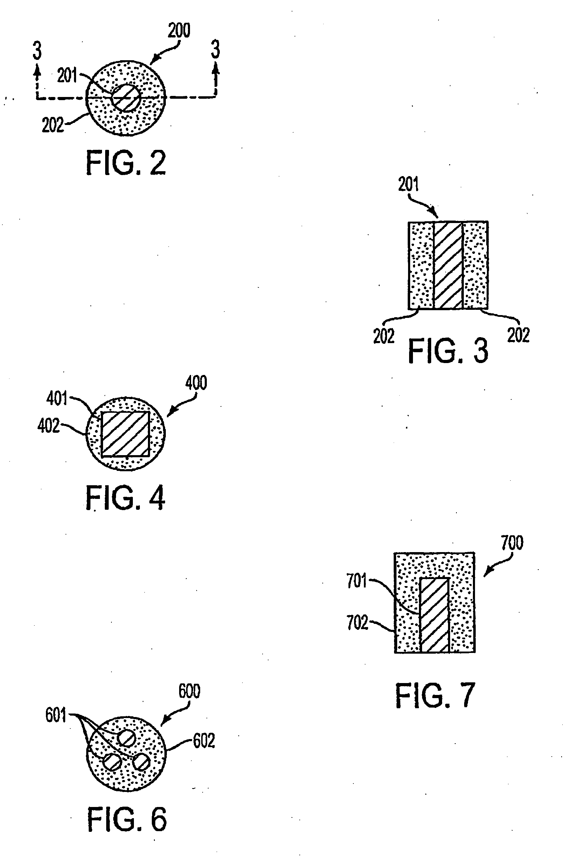

As shown in Figure 2, the substantially non-porous portion 201 of the preferred wick 200 is a cylindrical section and the porous portion/section 202 of wick 200 is a cylindrical ring-shaped section surrounding, and substantially concentric with, the non-porous portion 201. Figure 3 shows a cross-sectional view of the wick from Figure 2 in which non-porous portion 201 is surrounded on its longitudinal sides by the porous portion 202 and extends throughout the entire length of the wick 200, exposing the ends of the substantially non-porous portion 201 at both ends of the wick 200.

In one embodiment, a preferred cylinder comprising the substantially non-porous portion 201 of wick 200 has a length of about 0.5 to about 5 cm and a diameter of about 0.1 to about 2 cm. A preferred cylindrical ring shaped section comprising the porous portion 202 has a length of about 0.5 to about 5 cm and a diameter of about 0.2 to about 2.2 cm. A wick of this size is suitable for a preferred atomization device. However, the present invention is by no means limited by this size of wick and different wick sizes, both smaller and larger, are envisioned.

While a cylindrical substantially non-porous section extending completely through the wick and surrounded by a cylindrical ring-shaped porous section may be used, any one of a number of other shapes may be used for both the porous section of wick or the non-porous section of wick. For example, as shown in Figure 4, a wick 400 could comprise a substantially non-porous rectangular section 401 inside a cylindrical porous portion 402. Also, as shown in Figure 6, multiple substantially non-porous portions 601 may be dispersed in a porous portions 602 to form wick 600, or vice-versa.

In addition, as shown in Figure 7, the substantially non-porous portion 701 of wick 700, within the porous portion of wick 702, need not extend throughout the entire length of the wick so long as the substantially non-porous portion can reduce the flow rate in the manner desired, based on design preferences.

When such alternative designs are used, the preferred dimensions of the porous and substantially non-porous sections may be analyzed as the preferred percentages of cross-sectional areas for the respective portions. Specifically, preferred embodiments may be defined based on their respective cross-sectional areas of the wick (rather than their respective diameters), with the cross-section being taken in a plane substantially perpendicular to the length of the wick (i.e., direction of liquid migration). When the respective cross-sectional areas vary along the length of the wick, the measurement can be the average cross-sectional area.

In a preferred embodiment, the substantially non-porous section accounts for between about 20 to about 50% of the cross-sectional area (or average cross sectional area), and the porous section accounts for between 80 to 50%.

In one embodiment, the material for construction of the wick for both the porous and substantially non-porous section of the wick is preferably high density polyethylene. High density polyethylene was chosen because it can be easily manufactured to produce a wick with two sections meeting the size requirements for a wick in an atomizer device. Furthermore, high density polyethylene can produce a solid, dimensionally stable wick with the chosen porosities to vary the flow rate through the wick in the desired manner. While high density polyethylene is preferred, numerous other suitable materials may be used, including polypropylene, for example.

The preferred method of manufacturing a wick used in this embodiment, is a sintering process. A sintering process is preferred because of the ease of producing the desired characteristics of the wick in a cost effective manner. This process involves sintering inorganic or organic beads of, for example, a ceramic, PE, PP, HDPE, or the like. Preferably, the process involves a first step of sintering the core (typically, the nonporous section). Once the core is formed, the other section (e.g., porous section) is then sintered around the core. While a sintering process may be preferred, other manufacturing techniques, such as inserting a fully-formed substantially non-porous portion of wick into a fully-formed porous portion, may be used.

To control the flow rate of liquid through the wick to supply adequate liquid to the orifice plate and reduce flooding, the porous portion of wick has a preferred porosity of approximately sixty percent by volume and, ideally, the substantially non-porous portion of the wick has no porosity. However, as a practical matter, a section of wick with no porosity may be difficult to manufacture. Accordingly, the porosity of the inner portion of the wick is preferably less than about five percent by volume. A porosity of less than about five percent by volume produces a substantially non-porous section of wick and can provide the desired limitation of flow rate needed to reduce flooding of the orifice plate. While a porosity of approximately sixty percent by volume for the porous portion of wick and a porosity of less than about five percent by volume for the substantially non-porous portion of wick are preferred, these values may vary and other embodiments may contain different porosities.

While specific embodiments of the present invention have been illustrated and described, it will be apparent to those skilled in the art that various changes and modification may be made.

### INDUSTRIAL APPLICABILITY

The present invention provides an atomizer according to claim 1 with a wick with a controlled flow rate. We envision that this wick preferably can be used, for example, to transport liquid from a reservoir to an orifice plate in an atomization device for dispersing vapor into the ambient air while reducing flooding of the orifices plate. Since the wick reduces flooding of the orifice plate, this invention facilitates the continuous operation of an atomizer device that dispenses fragrances, insecticides, and any other vaporizable materials into the ambient air to freshen or deodorize the air or exterminate or repel unwanted pests.

## Claims

1. An atomizer device (34) comprising:
a vibratory orifice plate (37), which has a plurality of minute orifices formed therethrough, the vibratory orifice plate (37) being configured to vibrate so as to eject liquid, supplied to one side of the orifice plate (37), through the minute orifices;
a container (3) which contains the liquid to be supplied to the orifice plate;
and
an elongate wick (56) having a lower end which is immersed in the liquid within the container (3), and an upper end positioned above the container (31),
wherein, when the upper end of the wick (56) is brought into contact with the vibratory orifice plate (37), the wick (56) supplies the liquid from the container (31) to the vibratory orifice plate (37) for atomization;
whereby
the wick comprises a porous portion (202) having capillary passages for drawing the liquid from the lower end to the upper end, and a substantially non-porous portion (201) that has not more than 5% by volume porosity, wherein the porous portion (202) surrounds the substantially non-porous portion (201), and the substantially non-porous portion (201) of wick extends throughout the length of the entire wick; and the wick is formed by sintering inorganic or organic beads.

2. An atomizer device according to claim 1, wherein said substantially non-porous portion (601) of wick consists of multiple areas (601) of a cross-section of the wick.

3. An atomizer device according to claim 1, wherein said substantially non-porous (201) portion of wick is cylindrical in shape and said porous (202) portion of wick is cylindrical in shape.

4. An atomizer device according to any preceding claim, wherein said substantially non-porous portion (201) and said porous portion (202) are formed of the same material.

5. An atomizing device according to claim 1, wherein the material is a high-density polyethylene.

6. An atomizer device according to any preceding claim, wherein said porous portion (202) of wick has a porosity of sixty percent by volume or more.

7. An atomizer device according to any preceding claim wherein the substantially non-porous portion (201) occupies 20% to 50% of the cross-sectional area of the wick

## Patentansprüche

1. Zerstäubervorrichtung (34) mit:
einer vibrierenden Lochplatte (37), die eine Vielzahl von winzigen Öffnungen aufweist, die durch sie hindurch ausgebildet sind, wobei die vibrierende Lochplatte (37) ausgebildet ist, so zu vibrieren, dass sie eine Flüssigkeit durch die winzigen Öffnungen ausstößt, die einer der Seiten der Lochplatte (37) zugeführt wird;
einem Behälter (3), der die der Lochplatte zuzuführende Flüssigkeit enthält; und
einem länglichen Docht (56), der ein unteres Ende, das in die Flüssigkeit innerhalb des Behälters (3) eingetaucht ist, und ein oberes Ende aufweist, das oberhalb des Behälters (31) angeordnet ist, wobei der Docht (56) die Flüssigkeit aus dem Behälter (31) der vibrierenden Lochplatte zur Zerstäubung zuführt, wenn das obere Ende des Dochts (56) in Kontakt mit der vibrierenden Lochplatte (37) gebracht wird;
wobei der Docht einen porösen Abschnitt (202) umfasst, der Kapillardurchgänge aufweist, um Flüssigkeit von dem unteren Ende zu dem oberen Ende zu ziehen, und einen im Wesentlichen nicht-porösen Abschnitt (201) aufweist, der nicht mehr als 5 % Porosität bezogen auf das Volumen aufweist, wobei der poröse Abschnitt (202) den im Wesentlichen nicht-porösen Abschnitt (201) umgibt, und der im Wesentlichen nicht-poröse Abschnitt (201) des Dochts sich durchgehend über die Länge des gesamten Dochts erstreckt; und der Docht durch Sintern von nicht-organischen oder organischen Kügelchen hergestellt ist.

2. Zerstäubervornchtung nach Anspruch 1, wobei der im Wesentlichen nicht-poröse Abschnitt (601) des Dochts aus mehreren Bereichen (601) eines Querschnitts des Dochts besteht.

3. Zerstäubervorrichtung nach Anspruch 1, bei der der im Wesentlichen nicht-poröse (201) Abschnitt des Dochts eine zylindrische Form und der poröse (202) Abschnitt des Dochts eine zylindrische Form hat.

4. Zerstäubervorrichtung nach einem der vorhergehenden Ansprüche, bei der der im Wesentlichen nicht-poröse Abschnitt (201) und der poröse Abschnitt (202) aus dem gleichen Material hergestellt sind.

5. Zerstäubervorrichtung nach Anspruch 1, bei dem das Material ein Polyethylen hoher Dichte ist.

6. Zerstäubervornchtung nach einem der vorhergehenden Ansprüche, bei dem der poröse Abschnitt (202) des Dochts eine Porosität von 60 % oder mehr bezogen auf das Volumen hat.

7. Zerstäubervorrichtung nach einem der vorhergehenden Ansprüche, bei dem der im Wesentlichen nicht-poröse Abschnitt (201) 20-50 % der Querschnittsfläche des Dochts in Anspruch nimmt.

## Revendications

1. Dispositif d'atomiseur (34) comprenant :
une plaque à orifices vibratoire (37), qui comporte une pluralité d'orifices minuscules formés à travers elle, la plaque à orifices vibratoire (37) étant configurée pour vibrer de façon à éjecter du liquide, alimenté d'un côté de la plaque à orifices (37), à travers les orifices minute ;
un récipient (3) qui contient le liquide à alimenter vers la plaque à orifices ; et
une mèche allongée (56) ayant une extrémité inférieure qui est immergée dans le liquide dans le récipient (3), et une extrémité supérieure positionnée au-dessus du récipient (31),
dans lequel, quand l'extrémité supérieure de la mèche (56) est amenée en contact avec la plaque à orifices vibratoire (37), la mèche (56) alimente le liquide depuis le récipient (31) vers la plaque à orifices vibratoire (37) pour atomisation ;
moyennant quoi
la mèche comprend une partie poreuse (202) ayant des passages capillaires pour amener le liquide depuis l'extrémité inférieure vers l'extrémité supérieure, et une partie sensiblement non poreuse (201) qui n'a pas plus de 5% en volume de porosité, dans lequel la partie poreuse (202) entoure la partie sensiblement non poreuse (201), et la partie sensiblement non poreuse (201) de la mèche s'étend à travers la longueur de toute la mèche ; et la mèche est formée en frittant des particules inorganiques ou organiques.

2. Dispositif d'atomiseur selon la revendication 1, dans lequel ladite partie sensiblement non poreuse (601) de la mèche consiste en de multiples zones (601) d'une section transversale de la mèche.

3. Dispositif d'atomiseur selon la revendication 1, dans lequel ladite partie sensiblement non poreuse (201) de la mèche est de forme cylindrique et ladite partie poreuse (202) de la mèche est de forme cylindrique.

4. Dispositif d'atomiseur selon l'une quelconque des revendications précédentes, dans lequel ladite partie sensiblement non poreuse (201) et ladite partie poreuse (202) sont formées du même matériau.

5. Dispositif d'atomiseur selon la revendication 1, dans lequel le matériau est un polyéthylène à haute densité.

6. Dispositif d'atomiseur selon l'une quelconque des revendications précédentes, dans lequel ladite partie poreuse (202) de la mèche a une porosité de 60% en volume ou plus.

7. Dispositif d'atomiseur selon l'une quelconque des revendications précédentes, dans lequel la partie sensiblement non poreuse (201) occupe de 20% à 50% de l'aire en section transversale de la mèche.
